(19) Europäisches Patentamt
European Patent Office
Office européen des brevets

(11) Veröffentlichungsnummer: **0 270 491 A2**

(12) **EUROPÄISCHE PATENTANMELDUNG**

(21) Anmeldenummer: **87810668.1**

(22) Anmeldetag: **16.11.87**

(51) Int. Cl.[4]: **C 07 C 43/174**
C 07 C 43/168,
C 07 C 43/166, A 01 N 31/14

(30) Priorität: **20.11.86 CH 4658/86**
**01.10.87 CH 3825/87**

(43) Veröffentlichungstag der Anmeldung:
**08.06.88 Patentblatt 88/23**

(84) Benannte Vertragsstaaten:
**AT BE CH DE ES FR GB GR IT LI NL**

(71) Anmelder: **CIBA-GEIGY AG**
**Klybeckstrasse 141**
**CH-4002 Basel (CH)**

(72) Erfinder: **Gsell, Laurenz, Dr.**
**Maiengasse 56**
**CH-4056 Basel (CH)**

(54) **Substituierte Phenylbenzyl-(dimethyl-cyclopropylmethyl)-äther.**

(57) Neue substituierte Phenylbenzyl-(dimethyl-cyclopropylmethyl)-äther der Formel

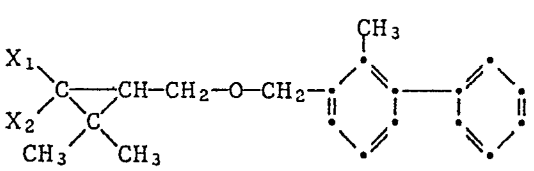

worin
$X_1$ und $X_2$ unabhängig voneinander Fluor, Chlor oder Brom; oder $X_1$ und $X_2$ gleichzeitig Methyl bedeuten, und die optischen Isomeren der Verbindung der Formel I; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten, speziell deren Larvalen Stadien. Die neuen Verbindungen weisen vor allem gute Wirksamkeit gegen pflanzenschädigende Insekten in Reiskulturen auf.

EP 0 270 491 A2

**Beschreibung**

Substituierte Phenylbenzyl-(dimethyl-cyclopropylmethyl)-äther

Die vorliegende Erfindung betrifft neuartige, substituierte (2-Methyl-3-phenylbenzyl)-(3,3-dimethylcyclopropylmethyl)-äther und deren optische Isomere, Verfahren zur Herstellung der neuartigen Verbindungen sowie ihre Verwendung in der Schädlingsbekämpfung.

Die neuen erfindungsgemässen Aether haben die Formel I

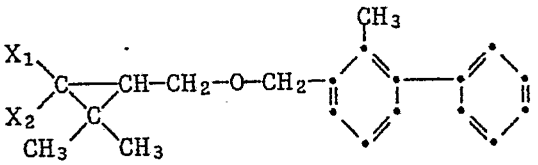

(I),

worin
$X_1$ und $X_2$ unabhängig voneinander Fluor, Chlor oder Brom; oder $X_1$ und $X_2$ gleichzeitig Methyl bedeuten. Die vorliegende Erfindung schliesst auch optische Isomeren von Verbindungen der Formel I ein.

Hervorzuheben wegen ihrer biologischen Wirkung sind Verbindungen der Formel I, worin $X_1$ und $X_2$ gleichzeitig Fluor oder Chlor oder Brom oder Methyl bedeuten. Von besonderem Interesse sind diejenigen Verbindungen der Formel I, worin $X_2$ Chlor bedeutet.

Die Verbindungen der Formel I können in an sich bekannter Weise hergestellt werden, indem man eine Verbindung der Formeln II oder IIa

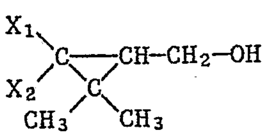

(II)

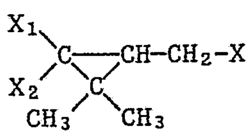

(IIa)

mit einer Verbindung der Formel III bzw. IIIa

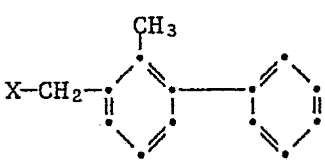

(III)

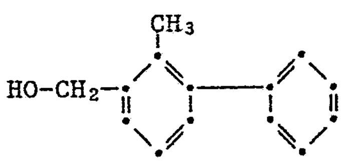

(IIIa)

umsetzt, wobei $X_1$ und $X_2$ die vorstehend angegebenen Bedeutungen besitzen und X für ein Halogenatom, vorzugsweise Brom oder Chlor, und im Falle der Verbindungen der Formeln IIa und III auch für die p-Toluolsulfonatgruppe steht.

Das obige Verfahren wird bei einer Reaktionstemperatur zwischen -10 und 120° C, zumeist zwischen 0 und 30° C, bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoff, insbesondere Benzol, Toluol, Xylole, Chloroform und Chlorbenzol; Nitrile, wie Acetonitril; Dimethylsulfoxid und Ketone wie Aceton und Methylketon sowie Hexan, Die bei dem Verfahren stattfindende Verätherung wird mit Vorteil in Gegenwart von Basen, wie Alkalihydroxyden oder -carbonaten, speziell aber Metallhydriden, z.B. Natriumhydrid, durchgeführt.

Verbindungen der Formel I, worin $X_1$ und $X_2$ Fluor, Chlor oder Brom bedeuten, insbesondere solche, worin $X_1$ und $X_2$ gleichzeitig Fluor, Chlor oder Brom, vorzugsweise Chlor, bedeuten, können auch erhalten werden, indem man in an sich bekannter Weise die Verbindung der Formel IV

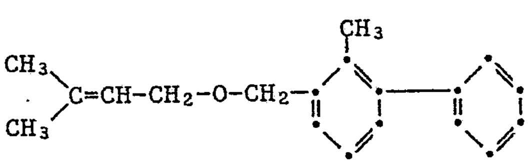

(IV) ,

vorzugsweise in einer phasentransfer-katalysierten Zweiphasenreaktion, mit einem Dihalogencarben, vorzugsweise Dichlorcarben, der Formel V

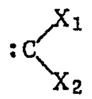 (V),

worin $X_1$ und $X_2$ Fluor, Chlor oder Brom bedeuten, umsetzt. Im Rahmen des vorliegenden Verfahrens kommen im Prinzip alle Methoden zur Erzeugung von Dihalogencarbenen in Betracht. Bei der erwähnten Zweiphasenreaktion bestehen die beiden Phasen aus der wässrigen Lösung einer starken Base, z.B. Natronlauge, und dem entsprechenden Haloform, z.B. Chloroform. Die Grenzflächenraktion wird durch Zusatz eines quaternären Ammoniumsalzes katalysiert [vgl. E.V. Dehmlow, Angew. Chem. 89, 521, (1977); W. E. Keller "Phase-Transfer-Reactions", Thieme-Verlag (1986)].

Die Ausgangsstoffe der Formeln II, IIa, III, IIIa und IV sind bekannt oder können analog bekannter Methoden hergestellt werden. So wird die Herstellung der substituierten Cyclopropylmethanole der Formel II in "Tetrahedron Letters" 34, 3331-35, beschrieben; die Herstellung dieses Verbindungstyps wird auch in Synthesis 1973, 112, und Helv. Chim. Acta 58, 2595 (1975) erwähnt. Die 2-Methyl-3-phenylbenzylderivate vom Typ der Formeln III und IIIa und deren Herstellung sind z.B. bekannt aus der US-Patentschrift No. 4 536 591.

Der neuartige Dimethylpropenyl-benzyläther der Formel IV kann in an sich bekannter Weise durch Umsetzung des Dimethylpropenols der Formel $(CH_3)_2C=CH-CH_2-OH$ oder des entsprechenden Bromids der Formel $(CH_3)_2C=CH-CH_2Br$ mit 2-Methyl-3-phenyl-benzylbromid bzw. -benzylalkohol erhalten werden, wobei diese Reaktionen zweckmässigerweise in Gegenwart von Natriumhydrid in Toluol//Dimethylformamid als Lösungsmittel durchgeführt werden.

Aus der EP-Patenschrift Nr. 120,238 sind bereits substituierte Dihalogendimethylcyclopropylmethyl-alkyläther als Mikrobizide bekannt, die sich strukturell von den erfindungsgemässen Verbindungen der Formel I vor allem durch das Fehlen der 2-Methyl-3-phenylbenzyl-Gruppierung unterscheiden.

Demgegenüber wurde nun gefunden, dass die neuartigen erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen. In diesem Zusammenhang wird auf die sehr geringe Fisch-Toxizität der erfindungsgemässen Verbindungen hingewiesen, ein für die Anwendung in Reis-Kulturen wichtiger Aspekt.

Insbesondere eignen sich die Verbindungen der Formeln I zur Bekämpfung von Insekten der Ordnungen Lepidoptera, Coleoptera, Homoptera, Heteroptera, Diptera, Thysanoptera, Orthoptera, Anoplura, Siphonaptera, Mallphaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Neben ihrer Wirkung genüber Mücken und Fliegen, wie z.B. Aëdes aegypti und Musca domestica, können Verbindungen der Formel I auch zur Bekämpfung von pflanzenschädigenden fressenden und saugenden Insekten in Zier- und Nutzpflanzungen, insbesondere in Reiskulturen (z.B. gegen Nilaparuata lugens und Nephotettix cincticeps) sowie in Getreide-, Baumwoll-, Obst- und Gemüsekulturen (z.B. gegen Laspeyresia pomonella, Leptinotarsa decemlineata, Epilachna varivestis, Spodoptera literalis und Heliothis virescens) eingesetzt werden. Die Verbindungen der Formel I zeichnen sich auch durch gute Wirkung gegen larvale Insektenstadien und Nymphen, insbesondere von fres senden Schadinsekten, aus. Auch können die Verbindungen der Formel I mit ausgezeichnetem Erfolg gegen pflanzenschädigende Zikaden, speziell in Reiskulturen, eingesetzt werden.

Die Verbindungen eignen sich ferner zur Bekämpfung von Ektoparasiten, z.B. Lucilia sericata, sowie von Zecken an Haus- und Nutz-tieren, z.B. durch Tier-, Stall- und Weidebehandlung.

Die Wirkung der erfindungsgemässen Verbindungen b.z.w. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, derekt versprühbaren oder verdünnbaren Lösungen, verdünnten Emulsionen, Spritzpulvern, löslichen Pulvern, Stäubemitteln, Granulaten, auch Verkapselungen in Z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprchend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder

Dioctylphthalat, aliphatische Kohlenwasserstoffe, wie Cyclohexan, Paraffine, Alkohole und Glykole sowie deren Aether und Ester, wie Aethanol. Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gestkeinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verkwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Koksnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr Helmut Stache "Tensid Taschenbuch", Carl Hanser Verlag München/Wien 1981.

Die pestiziden Zubereitungen enthalten - auf das Gewicht bezogen -in der Regl 0,1 bis 99%, insbesondere 0,1 bis 95%, eines Wirkstoffes der Formel I oder Kombinationen davon mit anderen Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

Herstellung des (2-Methyl-3-phenylbenzyl)-(2,2-dichlor-3,3-dimethylcyclopropylmethyl)-äthers

Es werden in einer Stickstoffatmosphäre 1,9 g 2,2-Dichlor-3,3-dimethylcyclopropylmethanol und 2,9 g 2-Methyl-3-phenylbenzylbromid, gelöst in 20 ml eines Gemisches aus Toluol und Dimethylformamid (1/1) unter Eiskühlung (0-5° C) zu 0,6 g Natriumhydrid (50%ige Dispersion in Mineralöl) in 20 ml Toluol/Dimethylformamid (1/1) tropfenweise hinzugefügt. Nach Abklingen der Reaktion wird der Ansatz während 12 Stunden bei Raumtemperatur ausgerührt, auf gesättigte Ammoniumchlorid-Lösung gegossen und mit Toluol extrahiert. Die vereinigten Toluol-Extrakte werden mit gesättigter Kochsalzlösung gewaschen, über $MgSO_4$ getrocknet und am Rotationsverdampfer eingeengt. Das erhaltene Rohprodukt wird an Kieselgel mit Hexan/-Toluol (3:1) chromatographiert. Die Titelverbindung der Formel

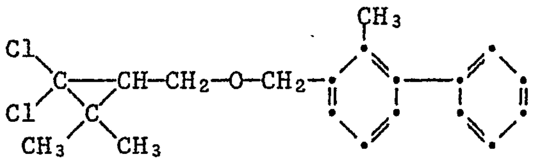

wird als klares Oel vom Brechungsindex $n_D^{24}$ = 1,5680 (Verbindung Nr. 1) erhalten.

Wie vorstehend beschrieben, werden auch die folgenden Verbindungen der Formel I hergestellt:

| Verbindung Nr. | $X_1$ | $X_2$ | physikal. Daten |
|---|---|---|---|
| 2 | $CH_3$ | $CH_3$ | $n_D^{24}$ = 1,5591 |
| 3 | Br | Cl | $n_D^{23}$ = 1,5602 |
| 4 | F | Cl | $n_D^{23}$ = 1,5491 |
| 5 | Br | Br | $n_D^{26}$ = 1,5907 |

Die folgenden Verbindungen der Formel I sind ebenfalls wie in Beispiel I beschrieben erhältlich:

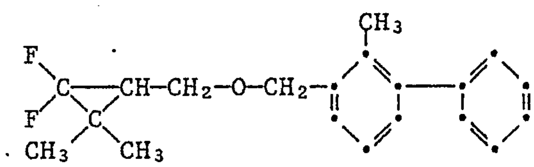

.

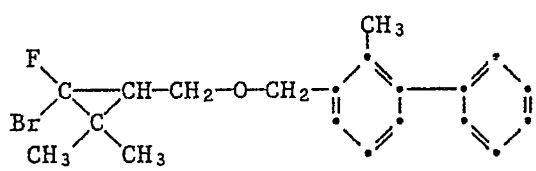

.

Beispiel 2:

Formulierungen für flüssige Wirkstoffe der Formel I gemäss Beispiel 1 oder Kombinationen dieser Wirkstoffe mit anderen Insektiziden oder Akariziden (% = Gewichtsprozent):

| 2.1. Emulsions-Konzentrate | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff resp. Wirkstoff-kombination | 80 % | 10 % | 5 % | 95 % |
| Aethylenglykolmonomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 2.3. Granulate | a) | b) |
|---|---|---|
| Wirkstoff resp. Wirkstoff-kombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4. Stäubemittel

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss Beispiel I oder Kombinationen dieser Wirkstoffe mit andern Insektiziden oder Akariziden (% = Gewichtsprozent):

2.5. Spritzpulver

| | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2.6. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

2.8. Extruder-Granulat

| | |
|---|---|
| Wirkstoff oder Wirkstoffkombination | 10 % |
| Na-Ligninsulfonat | 2 % |
| Carboxymethylcellulose | 1 % |

Kaolin 87 %

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

2.9. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

2.10. Suspensions-Konzentrat

Wirkstoff oder Wirkstoffkombination 40 %
Aethylenglykol 10 %
Nonylphenolpolyäthylenglykoläther
(15 Mol AeO) 6 %
Na-Ligninsulfonat 10 %
Carboxymethylcellulose 1 %
37 %ige wässrige Formaldehyd-Lösung 0,2 %
Silikonöl in Form einer 75 %igen
wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Verbindungen der Formel I gemäss Beispiel 1 zeigen gute Wirkung im obigen Test.

Beispiel 3:

Wirkung gegen Lucilia sericata (Larven)

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 4:

Kontaktwirkung auf Aphis craccivora

In Töpfen angezogene 4-5 Tage alte Bohnenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22° C und einer rel. Luftfeuchtigkeit von eta 55 % durchgeführt.

Die Verbindungen Nr. 1 und 2 gemäss Beispiel 1 zeigen 80-100 % Wirkung in diesem Test.

Beispiel 5:

Kontaktwirkung auf Myzus persicae

Etwa 4 bis 5 Tage alte, in Wasser angezogene Bohenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22° C und etwa 60 % relativer Luftfeuchtigkeit durchgeführt.

Verbindungen gemäss Beispiel 1 zeigen gute Wirkung in diesem Test.

Beispiel 6: Kontakt-Wirkung auf Laodelphax striatellus und Nilaparvata lugens (Nymphen);

Der Test wird an wachsenden Pflanzen durchgeführt. Dazu werden jeweils 8 Reispflanzen (Dicke des Stengels 4 mm) mit einer Höhe von ca. 20 cm in Töpfe (Durchmesser von 8 cm) eingepflanzt.

Die Pflanzen werden auf einem Drehteller mit 100 ml einer wässrigen Zubereitung enthaltend 400 ppm des jeweiligen formulierten Wirkstoffes besprüht. Nach dem Antrocknen des Spritzbelages erfolgt die Besiedlung jeder Pflanze mit je 20 Nymphen der Testtiere im dritten Stadium. Um die Zikaden am Entwichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des Adultstadiums über 6 Tage an der

behandelten Pflanze gehalten. Die Auswertung auf %-Mortalität erfolgt 6 Tage nach der Behandlung.

Die Verbindungen Nr. 1, 2, 4 und 5 gemäss Beispiel 1 zeigen 80-100 % Wirkung in diesem Test gegen Nilaparvate lugens.

Beispiel 7:

Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 400 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Die Verbindungen Nr. 1, 2 und 4 gemäss Beispiel 1 zeigen im obigem Test 80 - 100%ige Wirkung (Mortalität) gegen Nilaparvata lugens.

Beispiel 8:

Insektizide Frassgift-Wirkung

Ca. 25 cm hohe eingetopfte Baumwollpflanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis- bzw. Heliothis virescens-Larven im ersten larvalen Stadium besiedelt. Der Versuch wird bei 24°C und etwa 60 % relativer Luftfeuchtigkeit durchgeführt. Nach 120 Stunden wird die %-Mortalität der Test-Insekten gegenüber unbehandelten Kontrollen bestimmt.

Die Verbindungen Nr. 1 und 2 gemäss Beispiel 1 zeigen 80-100%ige Wirkung (Mortalität) gegen Spodoptera-Larven in diesem Test.

Beispiel 9:

Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Die erfindungsgemässen Verbindungen Nr. 1, 4 und 5 gemäss Beispiel 1 zeigen in diesem Test 80-100%ige Wirkung.

Beispiel 10:

Wirkung gegen Bodeninsekten (Diabrotica balteata)

Es werden 5 Maiskeimlinge von 1-3 cm Länge sowie eine Filterpapier-Rondelle in eine wässrige Wirkstofflösung enthaltend etwa 4 Vol.-% Aceton eingetaucht. Der Wirkstoffgehalt der verwendeten Lösung beträgt 400 ppm. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der Ansatz wird bei ca. 24°C und 40-60 % relativer Luftfeuchtigkeit und Tageslicht gehalten. Die Bonitur erfolgt nach 6 Tagen gegenüber unbehandelten Kontrollansätzen.

Die erfindungsgemässe Verbindung Nr. 1 zeigt in diesem Test eine Wirkung von 80-100 % (Mortalität).

Beispiel 11:

Wirkung gegen tierparasitäre Milben

Von mit Dermanyssus gallinae befallenen Hühnern werden Ansätze bestehend aus etwa 50 Milben verschiedener Stadien (Mischpopulation: Larven, Nymphen und Adulte) entnommen. Die Milbenansätze werden jeweils mit einer wässrigen Emulsion, Suspension bzw. Lösung enthaltend 400 ppm des zu prüfenden

Wirkstoffes benetzt. Hierzu werden die Milbenansätze in einem Reagenzglas mit der den Wirkstoff enthaltenden flüssigen Zubereitung übergossen; die Flüssigkeit wird anschliessend mit Hilfe eines Wattebausches aufgesogen. Die so benetzten und behandelten Milben verbleiben während 72 Stunden in dem Reagenzglas. Nach dieser Zeit wird die Abtötung der behandelten Milben gegenüber unbehandelten Kontrollansätzen ermittelt.

Verbindung Nr. 1 gemäss dem vorstehenden Beispiel 1 zeigt 100%ige Wirkung im obigen Test.

Beispiel 12: Wirkung gegen Zecken: Abtötungswirkung in verschiedenen Entwicklungsstadien

Als Testobjekte pro Ansatz werden nicht vollgesogene Larven (jeweils ca. 50), Nymphen (jeweils 5) oder Adulte (jeweils 5) der Zeckenarten Rhipicephalus bursa, Amblyomma hebraeum und Boophilus microplus verwendet. Die Testtiere (in der angegebenen Anzahl) werden für kurze Zeit in 2 bis 3 ml einer wässrigen Emulsion der zu untersuchenden Verbindung mit einer Konzentration von 400 ppm getaucht, die sich in einem Ragenzglas befindet. Die Reagenzgläser werden dann mit einem Wattepfropfen verschlossen und 10 Minuten nach dem Eintauchen der Testtiere geschüttelt. Dabei wird die Wirkstoff-Emulsion von den Wattepfropfen aufgesogen und die benetzten Test tiere in den so kontaminierten Reagenzgläsern belassen. Die Auswertung (% - Mortalität) erfolg für Larven nach 3 Tagen und für Nymphen und Adulte nach 14 Tagen.

Verbindung Nr. 1 gemäss dem vorstehenden Beispiel 1 zeigt im obigen Test 100%-ige Wirkung (Mortalität) gegen Nymphen und Adulte von Amblyomma hebraeum.

**Patentansprüche**

1. Verbindung der Formel 1

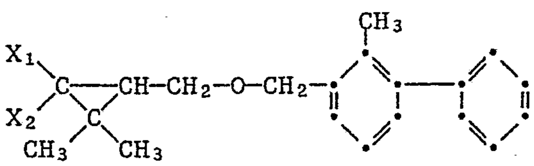

(I)

worin

$X_1$ und $X_2$ unabhängig voneinander Fluor, Chlor oder Brom; oder

$X_1$ und $X_2$ gleichzeitig Methyl bedeuten, und optische Isomeren der Verbindung der Formel I.

2. Verbindung gemäss Anspruch 1 der Formel I, dadurch gekennzeichnet, dass $X_1$ und $X_2$ gleichzeitig Fluor oder Chlor oder Brom oder Methyl bedeuten.

3. Verbindung gemäss Anspruch 1, dadurch gekennzeichnet, dass $X_2$ Chlor bedeutet.

4. Verbindung gemäss Anspruch 2 der Formel

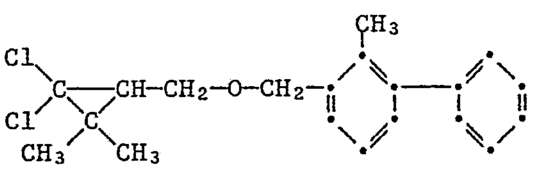

.

5. Verbindung gemäss Anspruch 3 der Formel

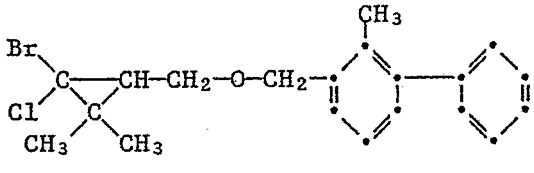

.

6. Verbindung gemäss Anspruch 3 der Formel

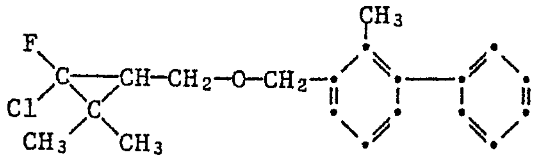

7. Verbindung gemäss Anspruch 2 der Formel

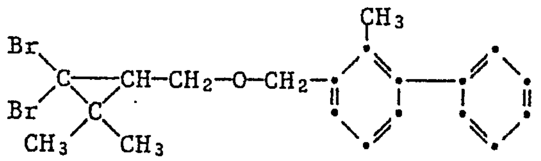

8. Verfahren zur Herstellung einer Verbindung der Formel I gemäss Anspruch 1, dadurch gekennzeichnet, dass man
eine Verbindung der Formel II oder IIa

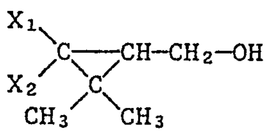 (II) 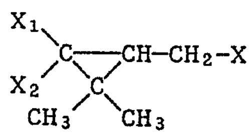 (IIa)

mit einer Verbindung der Formel III bzw. IIIa

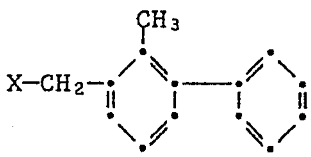 (III) 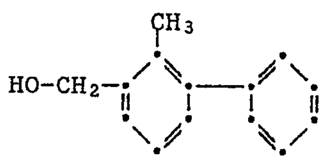 (IIIa)

umsetzt, wobei $X_1$ und $X_2$ die in Anspruch 1 angegebenen Bedeutungen besitzen und X für ein Halogenatom, vorzugsweise Brom oder Chlor, und im Falle der Verbindungen der Formeln IIa und III auch für die p-Toluolsulfonatgruppen steht.

9. Verfahren zur Herstellung einer Verbindung der Formel I gemäss Anspruch 1, worin $X_1$ und $X_2$ Fluor, Chlor oder Brom bedeuten, dadurch gekennzeichnet, dass man die Verbindung der Formel IV

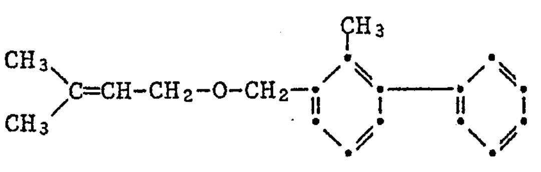 (IV)

mit einem Dihalogencarben der Formel V

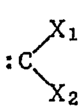 (V),

worin $X_1$ und $X_2$ Fluor, Chlor oder Brom bedeuten, umsetzt.

10. Verbindung der Formel IV gemäss Anspruch 9.

11. Schädlingsbekämpfungsmittel, welches als aktive Komponente eine Verbindung gemäss einem der Ansprüche 1 bis 7 zusammen mit geeigneten Trägern und/oder anderen Zuschlagstoffen enthält.

12. Verwendung einer Verbindung gemäss einem der Ansprüche 1 bis 7 zur Bekämpfung von Insekten und Vertretern der Ordnung Akarina an Tieren und Pflanzen.

13. Verwendung gemäss Anspruch 12 zur Bekämpfung von pflanzenschädigenden Insekten.

14. Verwendung gemäss Anspruch 13 zur Bekämpfung von pflanzenschädigenden Insekten in Reiskulturen.

15. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 6 oder mit einem Mittel enthaltend neben Zusatz- und Trägerstoffen eine pestizid wirksame Menge dieser Verbindung, in Kontakt bringt oder behandelt.